# FASCICULE DE BREVET EUROPEEN

(11) **EP 1 525 437 B1**
(45) Date de publication et mention de la délivrance du brevet: **21.06.2017**
(21) Numéro de dépôt: 03750809.0
(22) Date de dépôt: 15.07.2003
(51) Int. Cl.: G06M 1/22, G06M 1/24, G01F 11/00, A61M 15/00, G01F 11/28, G01F 11/30

(54) **INDICATEUR DE DOSES POUR DISPOSITIF DE DISTRIBUTION DE PRODUIT FLUIDE**
FLÜSSIGKEITSSPENDER MIT ZÄHLEINRICHTUNG DER DOSISEINHEITEN
DOSE INDICATOR FOR FLUID PRODUCT DISPENSING DEVICE

(30) Priorité: 29.07.2002 FR 0209617
(43) Date de publication de la demande: 27.04.2005
(73) Titulaire: Aptar France SAS, 27110 Le Neubourg (FR)
(72) Inventeur: STRADELLA, Fabio, I-16032 Camogli (IT); STRADELLA, Giuseppe, I-16032 Camogli (IT)
(74) Mandataire: CAPRI
(86) Numéro de dépôt international: PCT/FR2003/002233
(87) Numéro de publication internationale: WO 2004/013582

(56) Documents cités:
- EP-A- 0 254 391
- US-A- 5 421 482

## Description

La présente invention concerne un indicateur de doses, ainsi qu'un dispositif de distribution de produit fluide comportant un tel indicateur.

Dans le domaine des dispositifs de distribution de produit fluide destinés à distribuer plusieurs doses, et en particulier dans le domaine des pulvérisateurs, de nombreux systèmes destinés à indiquer le nombre de doses distribuées ou le nombre de doses restant à distribuer ont été développés.

La plupart de ces systèmes présentent de nombreux inconvénients. Ainsi, ils sont généralement conçus aux moyens de plusieurs roues dentées formant des engrenages, dont le nombre dépend de la quantité de doses à compter. Par conséquent, ces compteurs ou indicateurs peuvent devenir très complexes, encombrants, et donc coûteux à fabriquer et à assembler. D'autre part, l'indication est généralement donnée par des chiffres, qui sont souvent difficiles à lire pour l'utilisateur, en particulier lorsque les dispositifs de distribution sont destinés à distribuer un grand nombre de doses, par exemple jusqu'à 200 doses. De même, tous les systèmes de compteurs ou d'indication de doses actuels ne sont pas utilisables par les personnes ayant des problèmes de vue, et notamment par les aveugles. Un autre inconvénient majeur réside dans le fait que les compteurs existants nécessitent généralement une procédure d'assemblage du dispositif de distribution qui est modifiée de par la présence du compteur, et qui diffère donc de la procédure d'assemblage habituelle. Ceci augmente la complexité du dispositif, et implique par conséquent un coût supérieur.

Par ailleurs, la plupart des systèmes d'indication de doses sont disposés sur l'extrémité axiale du réservoir en étant perpendiculaire à l'axe de déplacement du réservoir lors de l'actionnement, ce qui implique un assemblage après mise en place du réservoir. Ceci augmente la complexité de l'assemblage et donc les coûts. Le document US-5 421 482 divulgue un tel système d'indication.

La présente invention a pour but de fournir un indicateur de doses destiné à un dispositif de distribution de produit fluide, qui ne reproduit pas les inconvénients susmentionnés.

En particulier, la présente invention a pour but de fournir un indicateur de doses qui est simple et peu coûteux à fabriquer et à assembler, et qui peut notamment être appliqué à tous les dispositifs de distribution de produit fluide existants sans impliquer une modification de la procédure d'assemblage.

La présente invention a aussi pour but de fournir un indicateur de doses qui est de petite dimension, indépendamment du nombre de doses contenu dans le dispositif de distribution.

La présente invention a également pour but de fournir un indicateur de doses qui forme une unité complète et séparée, et qui comprend notamment les moyens d'actionnement de l'indicateur.

La présente invention a aussi pour but de fournir un indicateur de doses qui soit facile à lire pour l'utilisateur, et qui peut aussi être utilisé par des personnes ayant des problèmes de vue, et notamment par les aveugles.

La présente invention a aussi pour but de fournir un indicateur de doses qui peut être assemblé avant la mise en place du réservoir.

La présente invention a encore pour but de fournir un indicateur de doses qui n'est pas disposé sur une extrémité axiale du réservoir.

La présente invention a donc pour objet un indicateur de doses pour dispositif de distribution de produit fluide tel que décrit dans la revendication 1. Des modes de réalisation avantageux, sont décrits dans les revendications dépendantes.

La présente invention a aussi pour objet un dispositif de distribution de produit fluide, comportant un réservoir de produit et un organe de distribution, tel qu'une pompe ou une valve, monté sur ledit réservoir, ainsi qu'un indicateur de doses tel que défini ci-dessus.

Avantageusement, l'indicateur de dose est actionné par une partie du réservoir qui est déplacée lors de l'actionnement du dispositif, et qui coopère avec un élément de transmission dudit indicateur.

D'autres caractéristiques et avantages de la présente invention apparaîtront plus clairement au cours de la description détaillée suivante d'un mode de réalisation particulier de celle-ci, faite en référence aux dessins joints, donnés à titre d'exemples non limitatifs, et sur lesquels,
- la figure 1 est une vue schématique de côté partiellement découpée d'un dispositif de distribution de produit fluide comportant un indicateur de doses selon un mode de réalisation avantageux de la présente invention,
- la figure 2 est une vue de face, similaire à celle de la figure 1,
- la figure 3 est une vue schématique en perspective d'une partie d'un indicateur de doses selon un mode de réalisation avantageux de la présente invention,
- la figure 4 est une vue éclatée d'une partie d'un indicateur de doses selon un mode de réalisation avantageux de l'invention,
- la figure 5 est une vue similaire à celle de la figure 4, prise selon un autre angle de vue, et
- la figure 6 est une vue schématique en section partiellement découpée d'une partie de l'indicateur des figures 3 à 5.

L'indicateur de doses A de la présente invention s'applique à tous types de dispositifs de distribution de produit fluide. Il s'applique toutefois plus particulièrement aux dispositifs de pulvérisation, et avantageusement aux dispositifs aérosols comportant une valve doseuse montée sur un récipient contenant un produit et un gaz propulseur.

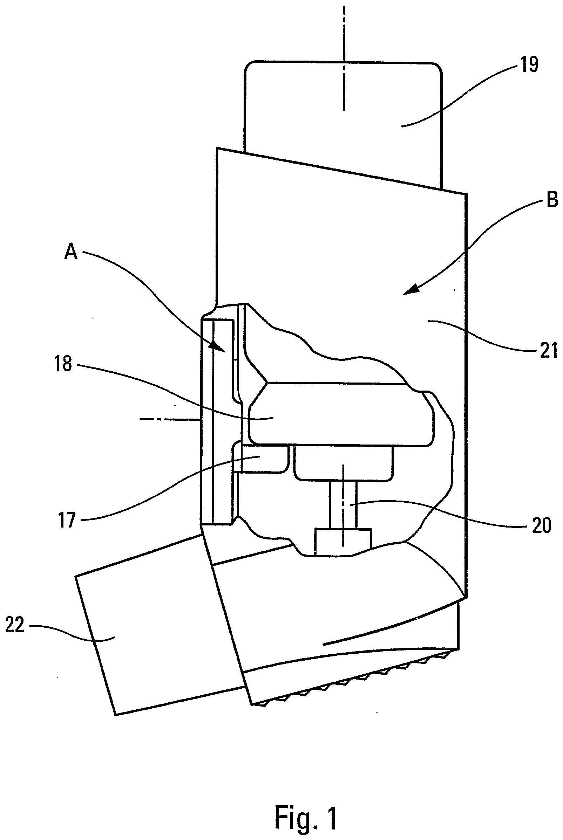

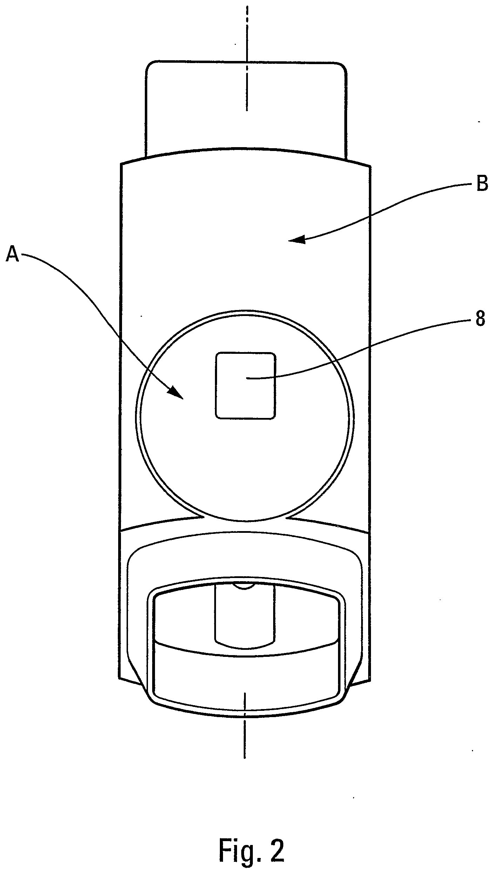

Les figures 1 et 2 représentent schématiquement un dispositif de distribution B auquel l'indicateur de doses A de la présente invention est particulièrement adapté. Ce dispositif comporte un corps 21 et un réservoir 19 sur lequel est assemblée une valve doseuse 20, l'actionnement du dispositif B étant obtenu par déplacement axial du réservoir 19 à l'intérieur du corps 21, ce déplacement entraînant une compression de la soupape de la valve 20 ce qui provoque l'expulsion d'une dose de produit à travers un orifice buccal 22. Bien entendu, la présente invention s'applique également à d'autres types de dispositifs de distribution, et notamment des dispositifs de pulvérisation du type nasal, ou des dispositifs comportant une pompe en lieu et place de la valve.

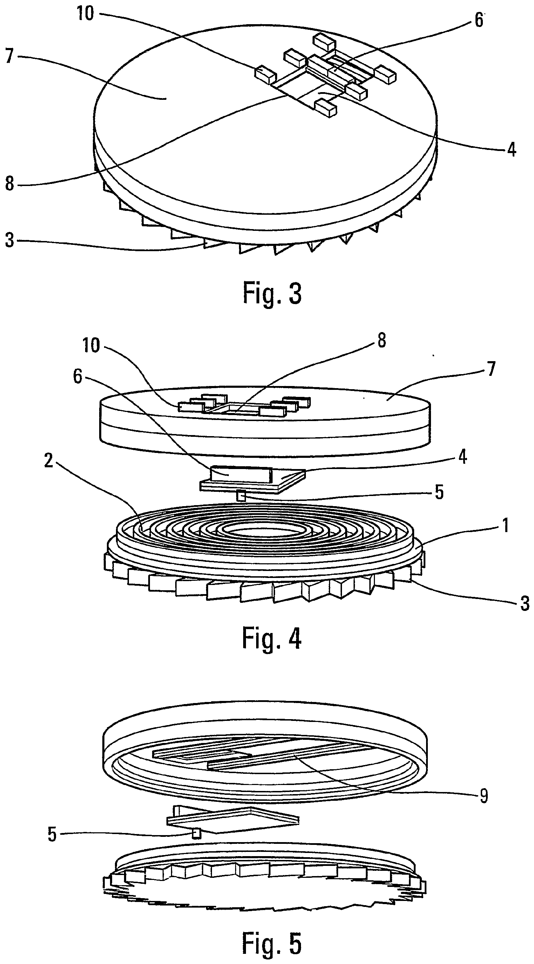

Les figures 3 à 6 représentent plus particulièrement un mode de réalisation de l'indicateur de doses A qui peut notamment être utilisé avec un dispositif de distribution de produit fluide B décrit ci-dessus. Cet indicateur de doses comprend un élément rotatif 1, réalisé sous la forme d'un disque rotatif, adapté à se déplacer en rotation autour d'un axe de rotation sensiblement perpendiculaire audit disque 1. Ce disque rotatif 1 est mince et pourvu d'un profil, de préférence en forme de spiral 2, qui peut avantageusement être réalisé au moyen d'une nervure. Le disque 1 comporte en outre une denture 3, réalisée sur sa périphérie, ladite denture 3 étant adaptée à coopérer avec des moyens d'actionnement qui sont adaptés à faire tourner ledit disque 1, et qui seront décrits plus amplement ci-après.

L'indicateur A représenté sur les figures comporte en outre un organe translatif 4, adapté à se déplacer en translation. Cet organe translatif 4 comporte une projection 5, ou tout autre moyen équivalent, qui coopère avec ledit profil en forme de spirale 2 du disque rotatif 1. En particulier, une rotation du disque rotatif 1 entraîne un déplacement de la projection 5 à l'intérieur du profil en spirale 2, ce qui provoque un déplacement en translation de l'organe translatif 4. Cet organe translatif 4 est de préférence réalisé sous la forme d'une plaque mince, qui comporte d'un côté des moyens d'indication 6 et de l'autre côté ladite projection 5 coopérant avec le disque rotatif 1.

Avantageusement, l'élément rotatif 1 et l'organe translatif 4 sont disposés dans un couvercle 7, qui est de préférence également de structure mince, et qui comporte une fenêtre de visualisation 8, avec laquelle les moyens d'indication 6 de l'organe translatif 4 coopèrent de manière à être visibles ou de manière à pouvoir être touchés par un utilisateur.

Les moyens de visualisation 6 peuvent être visuels et/ou tactiles. En ce qui concerne les moyens d'indication visuels, ceux-ci peuvent comporter des parties de couleurs différentes, de sorte qu'un déplacement en translation de ladite plaque mince 4 dans ladite fenêtre 8 provoque une répartition différente desdites couleurs dans ladite fenêtre. L'utilisateur peut ainsi visualiser le nombre de doses à distribuer, ou le nombre de doses déjà distribuées, en fonction de la répartition des couleurs dans ladite fenêtre. Soit l'organe translatif 4 est lui-même pourvu de parties de couleurs différentes, par exemple le vert et le rouge, soit la plaque translative 4 a une couleur définie, et le disque rotatif 1 disposé en dessous a une couleur différente, de sorte qu'au fur et à mesure que la plaque 4 se déplace en translation dans la fenêtre 8, le disque rotatif 1 de couleur différente apparaît progressivement dans ladite fenêtre, de sorte que l'utilisateur peut visualiser l'évolution de la distribution des doses. Avantageusement, l'invention prévoit également des moyens de visualisation tactiles, qui peuvent comporter des parties saillantes 6 prévues sur l'organe translatif 4, ainsi que des projections 10 qui peuvent être prévues autour de ladite fenêtre de visualisation 8, en étant intégrées par exemple dans le couvercle 7. Ainsi, l'utilisateur peut situer par toucher la position relative desdites parties saillantes mobiles 6 de l'organe translatif 4 par rapport auxdites projections fixes 10 dudit couvercle 7. Cette mise en oeuvre permet à une personne ayant des problèmes de vue, et notamment à une personne aveugle, de connaître la position de l'indicateur de doses, et donc le nombre de doses approximatif qui reste à distribuer ou qui ont déjà été distribuées. Bien entendu, ces moyens de visualisation tactiles et/ou visuels peuvent être réalisés de manière différente, les descriptions ci-dessus n'étant donnée qu'à titre d'exemples non limitatifs.

L'actionnement de l'indicateur A, et en particulier la rotation du disque rotatif 1 peut avantageusement être réalisé par des moyens d'actionnement intégrés dans ledit indicateur A. Ainsi, en référence aux figures 3 à 6, ces moyens d'actionnement comportent un élément d'entraînement 11 solidaire d'une bague 12 qui entoure ladite denture 3 du disque rotatif 1. Cet élément d'entraînement 11 est adapté à coopérer avec ladite denture 3 à chaque fois qu'une dose est distribuée. Avantageusement, des moyens anti-retour 13 sont prévus, notamment dans ladite bague 12, pour empêcher ledit disque rotatif 1 de tourner dans le sens inverse à celui qui lui est donné par l'élément d'entraînement 11 lors de l'actionnement.

Les moyens d'actionnement comportent également un élément de transmission 17 qui est adapté à coopérer avec le dispositif de distribution de produit fluide B, à chaque actionnement de celui-ci, ledit élément de transmission 17 coopérant d'autre part avec ledit élément d'entraînement 11 pour faire tourner ledit disque rotatif 1. En particulier, comme visible notamment sur la figure 1, ledit élément de transmission 17 est un épaulement solidaire de l'élément d'entraînement 11 et qui coopère avec une partie 18 du dispositif de distribution de produit fluide B qui est mobile pendant l'actionnement. Dans l'exemple représenté, il s'agit de la bague de fixation 18 de la valve doseuse 20 sur le réservoir 19. Bien entendu, et plus généralement, toute partie qui se déplace lors de l'actionnement du dispositif B est adaptée à coopérer avec l'épaulement 17 pour actionner l'indicateur de doses A.

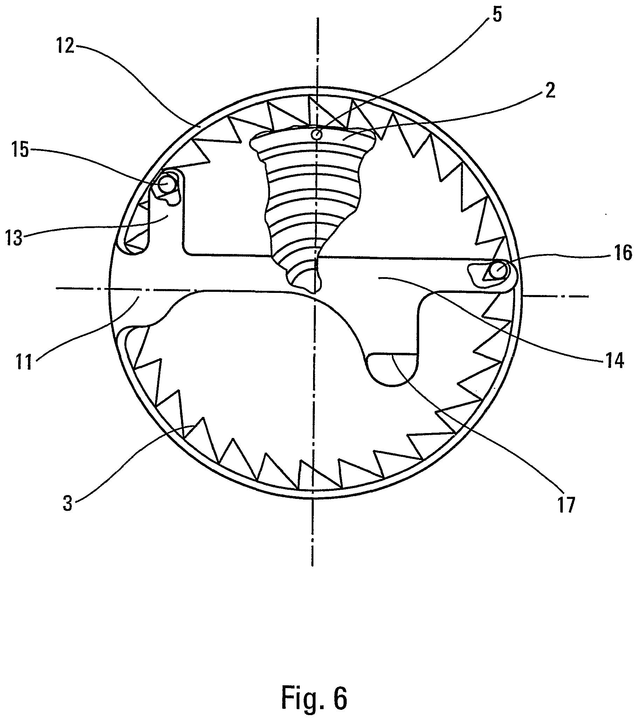

En référence à la figure 6, les moyens d'actionnement peuvent comporter une patte flexible 11 formant l'élément d'entraînement, cette patte flexible 11 pouvant être pourvue de deux parties flexibles 13 et 14 supportant chacune une dent 15 et 16 dont le fond est formé sous la forme d'un plan incliné. La patte flexible 11 supporte aussi ledit épaulement 17, et lorsque le dispositif de pulvérisation B est actionné, la bague de fixation 18 du réservoir entraîne la partie flexible 14 du bras 11 à se fléchir parallèlement au disque rotatif 1, ce qui provoque sa rotation au moyen de la dent 16 qui coopère avec la denture 3. Au même moment, la partie flexible 13 est forcée à se fléchir perpendiculairement au disque rotatif 1 par l'action de la denture 3 contre le fond incliné de la dent 15. Lorsque le réservoir 19 revient vers sa position de repos, la partie flexible 14 est libérée de sa charge et peut par conséquent se fléchir perpendiculairement au disque rotatif 1, grâce à l'action de la dent 16 avec la denture 3. De cette manière, la dent 16 vient en prise avec la dent suivante de la denture 3 et le dispositif est prêt pour le prochain actionnement. Evidemment, chaque rotation du disque rotatif 1 entraîne une translation de l'organe translatif 4 via la projection 5 de cet organe translatif qui coopère avec le profil en spirale 2 du disque rotatif 1.

Avantageusement, le couvercle 7 comporte des moyens de guidage 9, tels que des rails ou des nervures, qui coopèrent avec l'organe translatif 4 pour le guider dans son déplacement en translation.

Le nombre de dents sur la denture 3 et le nombre de spires du profil en spirale 2 du disque rotatif 1 donnent donc les caractéristiques de l'indicateur de doses, et notamment le nombre de doses que cet indicateur peut compter. Ainsi, dans l'exemple représenté sur la figure 6, il a été représenté trente dents dans la denture 3, et sept spires pour le profil en spirale 2. Par conséquent, l'indicateur de doses A représenté sur ces dessins est adapté à compter environ 210 doses. Bien entendu, ce nombre peut être varié à souhait en modifiant la structure du profil en spirale 2, ou en modifiant le nombre de dents sur la denture 3. La présente invention permet donc de réaliser des indicateurs de doses adaptés à compter un nombre quelconque de doses, sans modifier la géométrie ou la taille dudit indicateur. Comme déjà précisé précédemment, la structure du présent indicateur est particulièrement faible, notamment dans la dimension de l'épaisseur, et cet indicateur A peut donc très facilement être intégré dans les dispositifs de distribution de produit fluide B existants, comme cela est visible sur les figures 1 et 2.

En variante, le profil en spirale 2 du disque rotatif 1 pourrait comporter une partie finale avec un profil modifié. La partie finale de la spirale est la partie qui coopère avec la projection 5 de l'organe translatif 4 lorsque la dernière dose est comptée. Ce profil modifié pourrait être une courbure modifiée de la spirale, ou même une partie rectiligne, avec pour effet de modifier le déplacement en translation de l'organe translatif 4, et notamment de l'accélérer ou de l'augmenter en distance. Ainsi, l'utilisateur est informé que la dernière dose a été distribuée et que le dispositif est vide.

L'indicateur de doses de la présente invention permet de visualiser de manière simple et donc très peu coûteuse le nombre de doses distribuées ou le nombre de doses restant à distribuer dans le dispositif. La structure de l'indicateur est très mince, indépendamment du nombre de doses qu'il doit indiquer, et il ne comporte aucune partie saillante impliquant une modification du dispositif auquel il est appliqué. Comme visible sur la figure 1, l'indicateur de doses A de la présente invention s'applique très facilement à tous les dispositifs existants, sans que ceux-ci ne doivent être modifiés. La présence de l'indicateur A ne modifie pas non plus le processus d'assemblage du dispositif B. En particulier, l'indicateur peut être assemblé avant la mise en place du réservoir. L'indicateur peut par exemple être mis en place dans le dispositif B à travers une ouverture prévue à cet effet sur la partie frontale du corps 21 du dispositif. Un autre avantage du présent indicateur est que les moyens d'actionnement de l'indicateur sont intégrés dans celui-ci, de sorte que l'indicateur forme une unité autonome et séparée qui peut être pré-assemblée, et intégrée aisément dans n'importe quel dispositif de distribution de produit fluide, en particulier avant l'assemblage du réservoir. Comme également déjà expliqué précédemment, l'indicateur de la présente invention peut être utilisé par des personnes aveugles ou ayant des problèmes de vue, et la double indication visuelle et tactile garantit pour tous les utilisateurs une information fiable sur le nombre de doses distribuées ou restant à distribuer.

Bien entendu, la présente invention a été décrite en référence à un mode de réalisation particulier de celle-ci, représenté sur les dessins, mais elle n'est aucunement limitée à cette forme de réalisation particulière. Au contraire, un homme du métier peut y apporter toutes modifications sans sortir du cadre de la présente invention tel que défini dans les revendications annexées.

## Revendications

1. Indicateur de doses (A) pour dispositif de distribution de produit fluide (B), comportant un élément rotatif (1) déplaçable en rotation autour d'un axe de rotation et un organe translatif (4) déplaçable en translation dans une direction perpendiculaire audit axe de rotation, ledit élément rotatif (1) comportant un profil (2) coopérant avec une projection (5) dudit organe translatif (4), de telle sorte que chaque rotation dudit élément rotatif (1) entraîne une translation dudit organe translatif (4), la position dudit organe translatif (4) indiquant le nombre de doses distribuées ou restant à distribuer, ledit élément rotatif (1) étant un disque mince comportant une denture périphérique (3), ladite denture (3) coopérant avec des moyens d'actionnement (11, 17) adaptés à faire tourner ledit disque rotatif (1), lesdits moyens d'actionnement comportant un élément d'entraînement (11) coopérant avec ladite denture (3) chaque fois qu'une dose est distribuée, **caractérisé en ce que** ledit indicateur comporte une bague (12) entourant ladite denture (3), ledit élément d'entraînement (11) étant formé par une patte flexible solidaire de ladite bague (12), lesdits moyens d'actionnement comportant en outre un élément de transmission (17) formé par un épaulement solidaire dudit élément d'entraînement (11), coopérant avec une partie mobile (18) dudit dispositif de distribution de produit fluide (B) à chaque actionnement de celui-ci.

2. Indicateur selon la revendication 1, dans lequel ledit profil (2) est en forme de spirale.

3. Indicateur selon la revendication 1 ou 2, dans lequel ledit élément rotatif (1) et ledit organe translatif (4) sont disposés dans un couvercle (7) comportant une fenêtre de visualisation (8).

4. Indicateur selon la revendications 3, dans lequel l'élément rotatif (1), l'organe translatif (4), les moyens d'actionnement (11, 17) et le couvercle (7) forment une unité, qui peut être assemblée dans un dispositif de distribution de produit fluide (B).

5. Indicateur selon la revendication 3 ou 4, dans lequel ledit organe translatif (4) coulisse dans des moyens de guidage (9), tels que des nervures, prévus dans ledit couvercle (7).

6. Indicateur selon l'une quelconque des revendications précédentes, dans lequel ladite bague (12) comporte des moyens anti-retour (13), empêchant ledit disque rotatif (1) de tourner dans le sens opposé à celui induit par ledit élément d'entraînement (11).

7. Indicateur selon l'une quelconque des revendications précédentes, dans lequel ladite patte flexible formant ledit élément d'entrainement (11) est flexible parallèlement audit disque rotatif (1).

8. Indicateur selon l'une quelconque des revendications précédentes, dans lequel ledit organe translatif (4) comporte une plaque mince pourvue d'un côté de moyens d'indication (6) et de l'autre côté de ladite projection (5), lesdits moyens d'indication (6) coopérant avec une fenêtre de visualisation (8) visible et/ou adaptée à être touchée par l'utilisateur.

9. Indicateur selon la revendication 8, dans lequel lesdits moyens d'indication (6) sont visuels et/ou tactiles.

10. Indicateur selon la revendication 9, dans lequel lesdits moyens d'indication visuels comportent des parties de couleurs différentes, un déplacement en translation de ladite plaque mince (4) modifiant la répartition desdites couleurs dans ladite fenêtre de visualisation (8).

11. Indicateur selon la revendication 9 ou 10, dans lequel lesdits moyens d'indication tactiles comportent des parties saillantes (6) coopérant avec des projections (10) prévues autour de ladite fenêtre (8), permettant ainsi de situer par toucher la position relative desdites parties saillantes mobiles par rapport auxdites projections fixes (10).

12. Dispositif de distribution de produit fluide (B), comportant un réservoir de produit (19) et un organe de distribution, tel qu'une pompe ou une valve, monté sur ledit réservoir (19), **caractérisé en ce qu'**il comporte un indicateur de doses (A) selon l'une quelconque des revendications précédentes.

13. Dispositif selon la revendication 12, dans lequel l'indicateur de dose (A) est actionné par une partie (18) du réservoir (19) qui est déplacée lors de l'actionnement du dispositif (B), et qui coopère avec un élément de transmission (17) dudit indicateur (A).

14. Dispositif selon la revendication 12 ou 13, dans lequel l'axe de rotation de l'élément mobile (1) de l'indicateur de dose (A) est sensiblement perpendiculaire à la direction de déplacement du réservoir (19) lors de l'actionnement du dispositif.

## Patentansprüche

1. Dosen-Anzeigeeinrichtung (A) für eine Abgabevorrichtung (B) für ein fluidförmiges Produkt, die ein Drehelement (1), das um eine Rotationsachse verdrehbar ist, und ein Translationsorgan (4) umfasst, das in einer zur Drehachse senkrechten Richtung translatorisch verschiebbar ist, wobei das Drehelement (1) ein Profil (2) aufweist, das mit einem Vorsprung (5) des Translationsorgans (4) derart zusammenwirkt, dass jede Drehung des Drehelementes (1) eine Translationsbewegung des Translationsorgans (4) antreibt, wobei die Stellung des Translationsorgans (4) die Anzahl der abgegebenen oder der zur Abgabe verbleibenden Dosen anzeigt, wobei das Drehelement (1) eine dünne Scheibe ist, die eine Umfangszahnung (3) aufweist, wobei diese Zahnung (3) mit Betätigungseinrichtungen (11, 17) zusammenwirkt, die geeignet sind, die drehbare Scheibe (1) zu drehen, und wobei die Betätigungseinrichtungen ein Antriebselement (11) umfassen, das jedes Mal dann, wenn eine Dosis abgegeben wird, mit der Zahnung (3) zusammenwirkt, **dadurch gekennzeichnet, dass** die Anzeigeeinrichtung einen Ring (12) umfasst, der die Zahnung (3) umschließt, wobei das Antriebselement (11) von einer flexiblen, mit dem Ring (12) fest verbundenen Lasche gebildet ist, wobei die Betätigungseinrichtungen im Übrigen ein Übertragungselement (17) umfassen, das von einer Schulter gebildet ist, die mit dem Antriebselement (11) fest verbunden ist und mit einem beweglichen Teil (18) der Abgabevorrichtung (B) für das fluidförmige Produkt bei jeder Betätigung dieser Abgabevorrichtung zusammenwirkt.

2. Anzeigeeinrichtung nach Anspruch 1, bei der das Profil (2) spiralförmig ausgebildet ist.

3. Anzeigeeinrichtung nach Anspruch 1 oder 2, bei der das Drehelement (1) und das Translationsorgan (4) in einer Abdeckung (7) angeordnet sind, die ein Sichtfenster (8) aufweist.

4. Anzeigeeinrichtung nach Anspruch 3, bei der das Drehelement (1), das Translationsorgan (4), die Betätigungseinrichtungen (11, 17) und die Abdeckung (7) eine Einheit bilden, die in eine Abgabevorrichtung (B) für ein fluidförmiges Produkt eingebaut werden kann.

5. Anzeigeeinrichtung nach Anspruch 3 oder 4, bei der das Translationsorgan (4) in Führungseinrichtungen (9), wie zum Beispiel Rippen, gleitet, die in der Abdeckung (7) vorgesehen sind.

6. Anzeigeeinrichtung nach einem der vorhergehenden Ansprüche, bei welcher der Ring (12) eine rückwärts gerichtete Bewegung verhindernde Mittel (13) umfasst, welche die drehbare Scheibe (1) daran hindern, sich in der Richtung zu drehen, die der durch das Antriebselement (11) bewirkten Richtung entgegengesetzt ist.

7. Anzeigeeinrichtung nach einem der vorhergehenden Ansprüche, bei der die flexible Lasche, die das Antriebselement (11) bildet, parallel zu der besagten drehbaren Scheibe (1) angeordnet ist.

8. Anzeigevorrichtung nach einem der vorhergehenden Ansprüche, bei der das Translationsorgan (4) eine dünne Platte umfasst, die auf der einen Seite mit Anzeigeeinrichtungen (6) und auf der anderen Seite mit besagtem Vorsprung (5) versehen ist, wobei die Anzeigeeinrichtungen (6) mit einem Sichtfenster (8) zusammenwirken, das sichtbar und/oder geeignet ist, vom Verwender berührt zu werden.

9. Anzeigevorrichtung nach Anspruch 8, bei der die Anzeigeeinrichtungen (6) sichtbar und/oder berührbar sind.

10. Anzeigevorrichtung nach Anspruch 9, bei der die sichtbaren Anzeigeeinrichtungen Teile mit unterschiedlichen Farben aufweisen, so dass eine Translationsverschiebung der dünnen Platte (4) die Verteilung dieser Farben im Sichtfenster (8) verändert.

11. Anzeigevorrichtung nach Anspruch 9 oder 10, bei der die berührbaren Anzeigeeinrichtungen vorspringende Teile (6) umfassen, die mit Vorsprüngen (10) zusammenwirken, die um das Fenster (8) herum vorgesehen sind, um es zu ermöglichen, durch Berührung die relative Position der beweglichen vorspringenden Teile bezüglich der besagten feststehenden Vorsprünge (10) einzustellen.

12. Abgabevorrichtung (B) für ein fluidförmiges Produkt, die einen Produktbehälter (19) und ein Abgabeorgan, wie zum Beispiel eine Pumpe oder ein Ventil umfasst, das auf dem Behälter (19) montiert ist, **dadurch gekennzeichnet, dass** sie eine Dosen-Anzeigevorrichtung (A) nach einem der vorhergehenden Ansprüche umfasst.

13. Vorrichtung nach Anspruch 12, bei welcher die Dosen-Anzeigevorrichtung (A) durch einen Teil (18) des Behälters (19) betätigt wird, der während der Betätigung der Vorrichtung (B) verschoben wird und mit einem Übertragungselement (17) der Anzeigevorrichtung (A) zusammenwirkt.

14. Vorrichtung nach Anspruch 12 oder 13, bei welcher die Rotationsachse des beweglichen Elementes (1) der Dosen-Anzeigevorrichtung (A) im Wesentlichen senkrecht zu der Richtung verläuft, in welcher der Behälter (19) bei der Betätigung der Vorrichtung verschoben wird.

## Claims

1. A dose indicator (A) for a fluid dispenser device (B), said indicator including a rotary element (1) that is displaceable in rotation around a rotation axis and a slide member (4) that is displaceable in translation in a direction that is normal to said rotation axis, said rotary element (1) including a profile (2) co-operating with a projection (5) of said slide member (4), so that each rotation of said rotary element (1) causes said slide member (4) to be displaced in translation, the position of said slide member (4) indicating the number of doses dispensed or the number of doses still to be dispensed, said rotary element (1) being a thin disk including a set of peripheral teeth (3), said set of teeth (3) co-operating with actuator means (11, 17) designed to cause said rotary disk (1) to turn, said actuator means including a drive element (11) coming into co-operation with said set of teeth (3) each time a dose is dispensed, **characterized in that** said indicator comprises a ring (12) surrounding said set of teeth (3), said drive element (11) being formed by a flexible tab of said ring (12), said actuator means further comprising a transmission element (17) formed by a shoulder of said drive element (11), co-operating with a movable part (18) of said fluid dispenser device (B) each time said device is actuated.

2. An indicator according to claim 1, in which said profile (2) is a spiral-shaped profile.

3. An indicator according to claim 1 or claim 2, in which said rotary element (1) and said slide member (4) are disposed in a cover (7) including a display window (8).

4. An indicator according to claim 3, in which the rotary element (1), the slide member (4), the actuator means (11, 17), and the cover (7) form a unit which can be assembled in a fluid dispenser device (B).

5. An indicator according to claim 3 or claim 4, in which said slide member (4) slides in guide means (9), such as ribs, provided in said cover (7).

6. An indicator according to any preceding claim, in which said ring (12) includes anti-return means (13) preventing said rotary disk (1) from turning in the direction opposite to the direction in which it is turned by said drive element (11).

7. An indicator according to any preceding claim, in which said flexible tab forming said drive element (11) is flexible in a parallel direction with respect to said rotary disk (1).

8. An indicator according to any preceding claim, in which said slide member (4) comprises a thin plate provided on one side with indicator means (6), and on the other side with said projection (5), said indicator means (6) co-operating with a display window (8) so that said indicator means are visible and/or can be touched by the user.

9. An indicator according to claim 8, in which said indicator means (6) are visual and/or tactile.

10. An indicator according to claim 9, in which said visual indicator means include portions of different colors, a displacement in translation of said thin plate (4) modifying the distribution of said colors in said display window (8).

11. An indicator according to claim 9 or claim 10, in which said tactile indicator means include projecting portions (6) co-operating with projections (10) provided around said window (8), thereby enabling the positions of said moving projecting portions to be located relative to said fixed projections (10) by touch.

12. A fluid dispenser device (B), comprising a fluid reservoir (19) and a dispenser member, such as a pump or a valve, mounted on said reservoir (19), said device being **characterized in that** it further comprises a dose indicator (A) according to any preceding claim.

13. A device according to claim 12, in which the dose indicator (A) is actuated by a portion (18) of the reservoir (19) which is displaced while the device (B) is being actuated, and which co-operates with a transmission element (17) of said indicator (A).

14. A device according to claim 12 or claim 13, wherein the rotation axis of said rotary element (11) of the dose indicator (A) is substantially normal to the displacement direction of the reservoir (19) during actuation of the device.
